## Europäisches Patentamt
## European Patent Office
## Office européen des brevets

(11) Publication number: **0 075 992**
**B1**

# EUROPEAN PATENT SPECIFICATION

(45) Date of publication of patent specification: **18.09.85**

(51) Int. Cl.⁴: **A 61 K 9/14,** A 61 K 31/29

(21) Application number: **82201167.2**

(22) Date of filing: **21.09.82**

(54) Bismuth containing composition and method for the preparation thereof.

(30) Priority: **22.09.81 US 304372**

(43) Date of publication of application:
**06.04.83 Bulletin 83/14**

(45) Publication of the grant of the patent:
**18.09.85 Bulletin 85/38**

(84) Designated Contracting States:
**AT BE CH DE FR GB IT LI LU NL SE**

(56) References cited:
**GB-A-1 478 742**

**CHEMICAL ABSTRACTS, vol. 74, no. 23, 7th
June 1971, page 326, no. 130384s, Columbus
Ohio (USA);**

(73) Proprietor: **Gist - Brocades N.V.
Wateringseweg 1 P.O. Box 1
NL-2600 MA Delft (NL)**

(72) Inventor: **Bos, Petrus Johannes Hugo
Koninginnepad 38
NL-2635 JD Schipluiden (NL)**
Inventor: **Engel, Dirk Jacob Coenraad
Oosterduinweg 236
NL-2111 XK Aerdenhout (NL)**
Inventor: **de Jonge, Hayo
Fresialaan 27
NL-2106 BR Heemstede (NL)**

(74) Representative: **Van der Straaten, Jan Anthony
et al
c/o GIST-BROCADES N.V. Patents and
Trademarks Department Wateringseweg 1 P.O.
Box 1
NL-2600 MA Delft (NL)**

Courier Press, Leamington Spa, England.

## Description

This invention relates to bismuth containing solid compositions and to a method for their preparation. More particularly, this invention concerns a solid bismuth composition which easily dissolves in water giving a colloidal solution, its preparation and use in pharmaceutical preparations for treatment of peptic ulcer.

A bismuth containing solid composition has been described in British Patent No. 1,478,742. This known bismuth composition is obtained as a powder by spray drying a colloidal solution which is formed by solving in aqueous ammonia bismuth citrate, which is a water insoluble compound, and a polyhydric alcohol, usually a sugar e.g. sucrose, sorbitol or mannitol.

The above-mentioned colloidal solution of bismuth citrate, ammonia and polyhydric alcohol has been used for some time and is still in use as the active principle of a therapeutically effective liquid anti-ulcer drug.

The effectiveness of this colloidal bismuth composition was surprising in view of the inactivity of the starting materials, including in particular, solid bismuth citrate in treating peptic ulcers.

Though this drug is very active in healing ulcers, the liquid form has certain disadvantages. The ammoniacal smell is unpleasant for patients taking the liquid; moreover, the liquid is awkward to manage, especially outdoors. Therefore, a solid form of this drug would be very desirable. However, the colloidal solution, being an hydrophobic, inorganic colloid has been believed to be among the irreversible and irresoluble colloids. Since it was generally accepted by people skilled in the art, that such systems, i.e. inorganic hydrophobic colloids, always loose their colloidal properties during drying, for a long time no attempt was made to prepare a dry form of this drug.

Taking into account all previous experience with this type of hydrophobic colloidal systems, it was very surprising indeed to discover that the powder obtained in the above-described way and disclosed in British Patent 1,478,742 can be redissolved in water without any expedient to provide again a colloidal solution. The colloidal properties of the resultant solution are easily shown by light scattering (Tyndall effect). It is generally known that when a beam of light passes a colloidal solution, a part of the light is scattered sideways.

The resultant solid product has been shown to be as active against ulcers as the original colloidal solution.

It was further thought by people skilled in the art, that the colloidal solution of bismuth citrate, the spray-drying of which is described in the British patent mentioned above, must contain a compound which is able to stabilize the liquid colloidal system, to improve its taste and to increase its viscosity considerably. A polyhydric alcohol, preferably a sugar such as sucrose appeared to match all these requirements.

It has been believed that the above-mentioned phenomenon, i.e, ready reconstitution of a colloidal solution from the dried product of an inorganic hydrophobic colloid, previously unknown in the art of hydrophobic colloid chemistry, was the result of the large amount of solved sugar in the colloidal composition. The sugar was thought to prevent the clotting of the colloidal particles into larger insoluble aggregates, presumably due to a protecting layer with which each particle was thought to be coated during spray drying.

In order to stabilize hydrophobic colloids, it is known from prior art to apply compounds, often hydrophilic colloids, which like sugar have a strong interaction with the water structure, which compounds thus increase the viscosity of the colloidal system and lower the speed of flocculation. In fact, polyhydric alcohols, including sugars, are known to have a stabilizing effect on several colloidal systems and dispersions.

It was surprisingly found that a solution of bismuth citrate in aqueous ammonia can be dried satisfactorily in the absence of a polyhydric alcohol and surprisingly, the resultant powder is still able to solve colloidally in water. The powder thus obtained is, after combination with a polyhydric alcohol such as a sugar, as efficacious against peptic ulcers as a composition manufactured by spray-drying a solution in which the polyhydric alcohol is present.

In accordance with the present invention, a solid bismuth containing composition which is colloidally soluble in water is prepared by spray drying a colloidal solution of bismuth citrate in aqueous ammonia in the absence of polyhydric alcohol.

The solid product obtained by the described process is a feature of the invention. This product consists essentially of a complex of bismuth, citrate and hydroxyl ions and ammonia. The dry powder can be administered orally as such, but preferably it will be processed to a pharmaceutical composition or it may be dissolved in water to produce a palatable colloidal solution.

A particular advantage of the process of the invention is that in the absence of polyhydric alcohol, it is possible to spray dry at higher temperatures. In sugar containing powders decomposition occurs at temperatures over 160°C., which leads to caking of the powder. The walls of the drying chamber become covered with a sticky layer where the sugar further decomposes. This means that the yield of pharmaceutically acceptable powder diminishes. Further, after spray-drying, the cleaning of the chamber entails a lot of work. These disadvantages disappear when the sugar is not added to the solution of bismuth citrate in liquid ammonia but if desired, only to the spray-dried powder.

The temperature of the incoming air stream can now be raised to 210°C. and higher which means an increase in the drying capacity of at least 50%.

In carrying out the present process, the solution to be spray dried is fed to a conventional spray drier where it is sprayed, usually by means of a fast spinning rotor or by means of one or more nozzles and the resultant spray is contacted with an air stream which has been heated to a temperature of about 160—250°C., more preferably about 200—220°C. The temperature of the air stream at the outlet of the drier is about 80—120°C., more preferably 90—100°C.

To remove periodically or continuously possible deposits of spray-dried product on the walls of the drying chamber, the unit is provided with known means as are an installation which automatically knocks against the outerside of the chamber wall, or with an air broom within the chamber.

The colloidal solution is prepared by dissolving bismuth citrate in aqueous ammonia. Desirably up to about 44% (w/v) of bismuth citrate is dissolved in water, using enough ammonia to keep the bismuth salt in colloidal solution.

Preferably 0.2 to 2.0 g of ammonia per g. of bismuth citrate and more preferably 0.3 to 1.2 g of ammonia per g. of bismuth citrate is added and most preferably 0.9—1.1 g of ammonia per g of bismuth citrate is added.

The colloidal solution to be spray-dried contains bismuth citrate in an amount of up to 44% (w/v), more preferably in an amount of 16 to 30% (w/v) and most preferably in an amount of 20 to 25% (w/v) and ammonia in an amount of 2—33% (w/v), more preferably in an amount of 8 to 33% (w/v) and most preferably in an amount of 20—25% (w/v).

Additionally of about 0—45% potassium hydroxide and of about 0—40% citric acid to the solution to be spray-dried (one percentage at least should be greater than zero) gives extra protection against the formation of a precipitate. Preferably, about 30% of potassium hydroxide and about 17% of anhydrous citric acid are added. The percentages are by weight and based on the amount of bismuth citrate.

However, the amounts of added potassium hydroxide and citric acid cannot be varied fully independently of each other. Attention should be given to the pH of the initial colloidal solution which should be kept in the range of about 8—11.3, otherwise, a precipitate may be formed.

The concentration of the solution is such that the solid content is about 10 to 50% (w/v), preferably about 20—40% (w/v) and most preferably about 33% (w/v).

The spray dried product contains a complex of bismuth, citrate and hydroxyl ions and ammonia and may also contain residual water in an amount up to about 5% by weight. It has been found that compositions of the invention must contain at least 2% ammonia. If of ammonia less than 2% is present, the composition is not colloidally soluble in water and/or does not form a stable colloidal solution.

Preferably the solid composition of the invention contains not more than 6% of ammonia and about 32 to 52% by weight and more preferably about 39—42% by weight of bismuth calculated as $Bi_2O_3$ and 34—50% by weight and more preferably 38—47% by weight of citrate ions.

It has been found, surprisingly, that the spray dried product of the invention readily dissolves in water to reform a colloidal solution. No special expedient or additives are necessary to effect the dissolution of the solid bismuth composition of the invention; ordinary water at a pH of about 7 is all that is required to reconstitute a colloidal solution.

The invention also includes pharmaceutical compositions in dosage form for oral administration, such as sachets, capsules, a syrup, effervescent tablets or other oral tablets, e.g. chewing tablets, containing the therapeutically active dry bismuth preparation as the active ingredients. Polyhydric alcohols may be added to the dried or to a reconstituted aqueous colloidal solution, in an amount preferably less than 250% of the amount of bismuth citrate present in the starting solution. The polyhydric alcohol is selected from sucrose, maltose, fructose, glucose, manitol, sorbitol and glycerol. Sucrose is preferred.

The sucrose can be substituted by mannitol, which is less hygroscopic and less cariogenic, or by another polyhydric alcohol combined, if desired, with an artificial sweetener such as sodium saccharine or sodium cyclamate.

Also, some other expedients such as coloring and flavoring agents, and preservatives, may by preference be added after spraydrying.

The compositions may further contain pharmaceutically acceptable carriers.

The tablets may be formulated in the usual manner with one or more pharmaceutically acceptable diluents or excipients, for example, lactose or starch and include materials of a lubricating nature, for example, calcium stearate or magnesium stearate. Capsules made of absorbable materials, such as gelatin, may contain the active substance alone or in admixture with a solid or liquid diluent.

The compositions according to the invention in solid form or in the form of a reconstituted aqueous colloidal solution are therapeutically effective in the treatment of peptic ulcer, including gastric, duodenal and post-operative ulcer and peptic ulcer associated with hiatus hernia.

Suitable daily dosages for adult humans contain bismuth corresponding to 450—1000 mg. of $Bi_2O_3$. The dosage for children will depend on their weight and age and may be calculated by methods commonly used in medical practice. The daily dose for children under 10 years will correspond to 150—400 mg. of $Sl_2O_3$.

The pharmaceutical compositions in dosage forms therefore preferably have a bismuth content equivalent to 35—250 mg. of $Bi_2O_3$.

The invention is further illustrated by the following examples:

Example 1

  180.360 kg. bismuth citrate
  180.360 kg. ammonia (25%)
   31.170 kg. citric acid monohydrate
   52.120 kg. potassium hydroxide (85% pure)

are solved in water. The solution is diluted until the concentration of solids is 33% (w/v) and is then fed to a spray-drying unit which has been preheated by hot air. The unit has evaporative capacity of about 450 kg. of water per hour. The bismuth citrate solution is atomized by means of a fast spinning rotor. At the same time the unit is supplied with an air stream heated at a temperature of 200—220°C. This air dries the spray and carries the powder from the drying unit to at least one cyclone where the power is separated from the air. At the outlet of the drying unit the temperature of the air stream has fallen to 90—100°C. After leaving the drying chamber, the stream is mixed with cooler air, so that the dried powder collected in the last cyclone is at or about ambient temperature.

The unit is provided with an installation which automatically knocks against the outside of the chamber to remove possible deposits on the walls.

The products obtained from a series of runs carried out in the foregoing manner have been analyzed for bismuth, ammonia and water with the following results:

| | |
|---|---|
| Citrate | 38—47% |
| $Bi_2O_3$ | 39—42% |
| $NH_3$ | 2—6% |
| $H_2O$ | 5% or less |
| pH | 6—8 |

percentages except $H_2O$ relate to dry matter only.

Example 2

  63.4 kg bismuth citrate
  22.6 kg ammonia (25%)
   5.95 kg citric acid monohydrate
  18.2 kg potassium hydroxide (85% pure)

are solved in water. The solution is treated as described in example 1, and the power obtained shows the desired characteristics.

Example 3

  63.4 kg bismuth citrate
  45.3 kg ammonia (25%)
  11.9 kg citric acid monohydrate
   9.1 kg potassium hydroxide (85% pure)

are solved in water. The solution undergoes the treatment described in example 1 which yields a power with the desired characteristics.

Example 4

  Using known pharmaceutical techniques, tablets are prepared, containing 450 mg. of the spray-dried product prepared according to Examples 1—3, and further

  900 mg. of mannitol
   10 mg. of Aerosil® 200 (purified silicum dioxide)
  100 mg. of corn starch
   10 mg. of magnesium stearate
    1 mg. of sodium saccharine

The invention also includes within its scope the preparation of an aqueous solution from the dry powder. For instance, a solution suitable for oral administration may be obtained by dissolving 200 g. of the powder prepared according to Example 1 in water to a volume of 1 liter. Other physiologically acceptable substances may be added, for instance, to produce a desired pH or to improve the taste of the solution.

**Claims for the Contracting States: BE, CH, DE, FR, GB, IT, LI, LU, NL, SE**

1. A solid bismuth containing composition useful for treating peptic ulcers which is colloidally soluble in water at a pH of about 7, consisting essentially of a complex of bismuth, citrate and hydroxyl ions and ammonia, wherein said ammonia is present in a minimal amount of about 2% by weight, characterised by the absence of a polyhydric alcohol.

2. A solid bismuth containing composition according to claim 1, wherein ammonia is present in a maximal amount of about 6% by weight.

3. A solid bismuth composition according to claim 1 or 2 in which bismuth, calculated as $Bi_2O_3$ is present in an amount of about 39—42% by weight.

4. A process for the preparation of a solid bismuth containing composition according to any of claims 1 to 3 by spray drying a colloidal solution of bismuth citrate in aqueous ammonia, characterised in that the solution to be spray dried does not contain a polyhydric alcohol.

5. A process according to claim 4 which is carried out at a temperature of about 200—220°C.

6. A process according to claim 4 or 5 in which the colloidal solution contains 0.2 to 2 gram ammonia per gram of bismuth citrate.

7. A process according to claim 6 in which the colloidal solution contains 0.3 to 1.2 gram ammonia per gram of bismuth citrate.

8. A process according to claim 7 in which the colloidal solution contains 0.9—1.1 gram ammonia per gram of bismuth citrate.

9. A process according to claims 4—8 in which the colloidal solution contains up to 44% (w/v) of bismuth citrate.

10. A process according to claim 9 in which the colloidal solution contains 16 to 30% (w/v) of bismuth citrate.

11. A process according to claim 10 in which the colloidal solution contains (20—25% (w/v) of bismuth citrate.

12. A process according to claims 4, 5, 6 or 9 in which the colloidal solution contains 2—33% (w/v) of ammonia.

13. A process according to claims 4, 5, 6 or 9 in which the colloidal solution contains 8—33% (w/v) of ammonia.

14. A process according to claims 4, 5, 6 or 9 in which the colloidal solution contains 20—25% (w/v) of ammonia.

15. A pharmaceutical composition containing a composition defined in claim 1, 2 or 3 in an amount effective for the treatment of peptic ulcers in combination with a pharmaceutically acceptable carrier.

16. A process for preparing a pharmaceutical composition according to claim 15, characterised in that a solid bismuth containing composition according to claim 1, 2 or 3 in an amount effective for the treatment of peptic ulcer is combined with a pharmaceutically acceptable carrier.

17. A process according to claim 16 in which a polyhydric alcohol is added to said composition.

**Claims for the Contracting State: AT**

1. A process for the preparation of a solid composition which is colloidally soluble in water, consisting essentially of a complex of bismuth, citrate and hydroxyl ions and ammonia, wherein said ammonia is present in a minimal amount of about 2% by weight, by spray-drying a colloidal solution of bismuth citrate in aqueous ammonia, characterised in that the solution to be spray dried does not contain a polyhydric alcohol.

2. A process according to claim 1, wherein ammonia is present in the solid composition in a maximal amount of about 6% by weight.

3. A process according to claim 1 or 2 which is carried out at a temperature of about 200—220°C.

4. A process according to claim 1, 2 or 3 in which the colloidal solution contains 0.2 to 2 gram ammonia per gram of bismuth citrate.

5. A process according to claim 4 in which the colloidal solution contains 0.3 to 1.2 gram ammonia per gram of bismuth citrate.

6. A process according to claim 5 in which the colloidal solution contains 0.9—1.1 gram ammonia per gram of bismuth citrate.

7. A process according to claims 1—6 in which the colloidal solution contains up to 44% (w/v) of bismuth citrate.

8. A process according to claim 7 in which the colloidal solution contains 16 to 30% (w/v) of bismuth citrate.

9. A process according to claim 8 in which the colloidal solution contains 20—25% (w/v) of bismuth citrate.

10. A process according to claims 1, 2, 3, 4 or 7 in which the colloidal solution contains 2—33% (w/v) of ammonia.

11. A process according to claim 10 in which the colloidal solution contains 8—33% (w/v) of ammonia.

12. A process according to claim 11 in which the colloidal solution contains 20—25% (w/v) of ammonia.

13. A process for preparing a pharmaceutical composition which comprises combining the solid bismuth containing composition obtained according to any of the claims 1—12 in an amount effective for the treatment of ulcers with a pharmaceutically acceptable carrier.

14. A process for the preparation of a pharmaceutical composition according to claim 13 in which a polyhydric alcohol is added to said composition.

**Patentansprüche für den Vertragsstaaten: BE, CH, DE, FR, GB, IT, LI, LU, NL, SE**

1. Eine feste, Wismut enthaltende Zusammensetzung, brauchbar für die Behandlung von Magengeschwüren, die bei einem pH-Wert bon etwa 7 in Wasser kolloidal löslich ist, bestehend im wesentlichen aus einem Komplex von Wismut, Citrat und Hydroxylionen und Ammoniak, in welchem das Ammoniak in einer Mindestmenge von etwa 2 Gew.-% vorhanden ist, gekennzeichnet durch die Abwesenheit eines mehrwertigen Alkohols.

2. Eine feste, Wismut enthaltende Zusammensetzung nach Anspruch 1, in der Ammoniak in einer maximalen Menge von etwa 6 Gew.-% vorhanden ist.

3. Eine feste Wismutzusammensetzung nach Anspruch 1 oder 2, in der Wismut, berechnet als $Bi_2O_3$, in einer Menge von etwa 39 bis 42 Gew.-% vorhanden ist.

4. Ein Verfahren zur Herstellung einer festen, Wismut enthaltenden Zusammensetzung nach einem der Ansprüche 1 bis 3, durch Sprühtrocknen einer kolloidalen Lösung von Wismutcitrat in wässerigem Ammoniak, dadurch gekennzeichnet, daß die sprühzutrocknende Lösung keinen mehrwertigen Alkohol enthält.

5. Ein Verfahren nach Anspruch 4, das bei einer Temperatur von etwa 200 bis 220°C durchgeführt wird.

6. Ein Verfahren nach Anspruch 4 oder 5, bei dem die kolloidale Lösung 0,2 bis 2 g Ammoniak pro g Wismutcitrat enthält.

7. Ein Verfahren nach Anspruch 6, bei dem die kolloidale Lösung 0,3 bis 1,2 g Ammoniak pro g Wismutcitrat enthält.

8. Ein Verfahren nach Anspruch 7, bei dem die kolloidale Lösung 0,9 bis 1,1 g Ammoniak pro g Wismutcitrat enthält.

9. Ein Verfahren nach den Ansprüchen 4 bis 8, bei dem die kolloidale Lösung bis zu 44% (Gew./Vol.) Wismutcitrat enthält.

10. Ein Verfahren nach Anspruch 9, wobei die kolloidal Lösung 16 bis 30% (Gew./Vol.) Wismutcitrat enthält.

11. Ein Verfahren nach Anspruch 10, wobei die kolloidale Lösung 20 bis 25% (Gew./Vol.) Wismutcitrat enthält.

12. Ein Verfahren nach den Ansprüchen 4, 5, 6 oder 9, wobei die kolloidal Lösung 2 bis 33% (Gew./Vol.) Ammoniak enthält.

13. Ein Verfahren nach den Ansprüchen 4, 5, 6 oder 9, wobei die kolloidale Lösung 8 bis 33% (Gew./Vol.) Ammoniak enthält.

14. Ein Verfahren nach den Ansprüchen 4, 5, 6 oder 9, wobei die kolloidale Lösung 20 bis 25% (Gew./Vol.) Ammoniak enthält.

15. Eine pharmazeutische Zusammensetzung, enthaltend eine in Anspruch 1, 2 oder 3 definierte Zusammensetzung in einer für die Behandlung von Magengeschwüren wirksamen Menge in Kombination mit einem pharmazeutisch annehmbaren Träger.

16. Ein Verfahren zur Herstellung einer pharmazeutischen Zusammensetzung nach Anspruch 15, dadurch gekennzeichnet, daß eine feste, Wismut enthaltende Zusammensetzung gemäß Anspruch 1, 2 oder 3 in einer für die Behandlung von Magengeschwüren wirksamen Menge mit einem pharmazeutisch annehmbaren Träger kombiniert wird.

17. Verfahren nach Anspruch 16, bei dem der angeführten Zusammensetzung ein mehrwertiger Alkohol zugesetzt wird.

**Patentansprüche für den Vertragsstaat: AT**

1. Ein Verfahren zur Herstellung einer festen Zusammensetzung, die in Wasser kolloidal löslich ist, bestehend im wesentlichen aus einem Komplex von Wismut, Citrat und Hydroxylionen und Ammoniak wobei das Ammoniak in einer Mindestmenge von etwa 2 Gew.-% vorhanden ist, durch Sprühtrocknung einer kolloidalen Lösung von Wismutcitrat in wässerigem Ammoniak, dadurch gekennzeichnet, daß die sprühzutrocknende Lösung nicht einen mehrwertigen Alkohol enthält.

2. Ein Verfahren nach Anspruch 1, wobei Ammoniak in der festen Zusammensetzung in einer Höchstmenge von etwa 6 Gew.-% vorhanden ist.

3. Ein Verfahren nach Anspruch 1 oder 2, das bei einer Temperatur von etwa 200 bis 220°C durchgeführt wird.

4. Ein Verfahren nach Anspruch 1, 2 oder 3, wobei die kolloidale Lösung 0,2 bis 2 g Ammoniak pro g Wismutcitrat enthält.

5. Ein Verfahren nach Anspruch 4, wobei die kolloidale Lösung 0,3 bis 1,2 g Ammoniak pro g Wismutcitrat enthält.

6. Ein Verfahren nach Anspruch 5, wobei die kolloidale Lösung 0,9 bis 1,1 g Ammoniak pro g Wismutcitrat enthält.

7. Ein Verfahren nach den Ansprüchen 1 bis 6, wobei die kolloidale Lösung bis zu 44% (Gew./Vol.) Wismutcitrat enthält.

8. Ein Verfahren nach Anspruch 7, wobei die kolloidale Lösung 16 bis 30% (Gew./Vol.) Wismutcitrat enthält.

9. Ein Verfahren nach Anspruch 8, wobei die kolloidale Lösung 20 bis 25% (Gew./Vol.) Wismutcitrat enthält.

10. Ein Verfahren nach den Ansprüche 1, 2, 3, 4 oder 7, wobei die kolloidale Lösung 2 bis 33% (Gew./Vol.) Ammoniak enthält.

11. Ein Verfahren nach Anspruch 10, wobei die kolloidale Lösung 8 bis 33% (Gew./Vol.) Ammoniak enthält.

12. Ein Verfahren nach Anspruch 11, wobei die kolloidale Lösung 20 bis 25% (Gew./Vol.) Ammoniak enthält.

13. Ein Verfahren zur Herstellung einer pharmazeutischen Zusammensetzung, bei welchem die feste, Wismut enthaltende Zusammensetzung, die gemäß einem der Ansprüche 1 bis 12 erhalten wurde, in einer für die Behandlung von Magengeschwüren wirksamen Menge mit einem pharmazeutisch annehmbaren Träger komobiniert wird.

14. Ein Verfahren zur Herstellung einer pharmazeutischen Zusammensetzung nach Anspruch 13, wobei dieser Zusammensetzung ein mehrwertiger Alkohol zugesetzt wird.

**Revendications pour les Etats Contractants: BE, CH, DE, FR, GB, IT, LI, LU, NL, SE**

1. Composition solide contenant du bismuth utilie pour le traitement des ulcères peptiques, qui est colloïdalement soluble dans l'eau à un pH d'environ 7, consistant essentiellement en un complexe d'ions bismuth, citrate et hydroxyle et en ammonica, dans laquelle l'ammoniac est présent en une quantité minimale d'environ 2% en poids, caractérisée par l'absence d'un alcool polyhydroxylé.

2. Composition solide contenant du bismuth suivant la revendication 1, dans laquelle l'ammoniac est présent en une quantité maximale d'environ 6%.

3. Composition solide contenant du bismuth suivant la revendication 1 ou 2, dans laquelle le bismuth, calculé en $Bi_2O_3$, est présent en une quantité d'environ 39—42% en poids.

4. Procédé de préparation d'une composition solide contenant du bismuth suivant l'une quelconque des revendications 1 à 3, par séchage par pulvérisation d'une solution colloïdale de citrate de bismuth dans de l'ammoniaque aqueuse, caractérisé en ce que la solution à sécher par pulvérisation ne contient pas d'alcool polyhydroxylé.

5. Procédé suivant la revendication 4, qui est exécuté à une température d'environ 200—220°C.

6. Procédé suivant la revendication 4 ou 5, dans lequel la solution colloïdale contient 0,2 à 2 g d'ammoniaque par gramme de citrate de bismuth.

7. Procédé suivant la revendication 6, dans lequel la solution colloïdale contient 0,3 à 1,2 g d'ammoniaque par gramme de citrate de bismuth.

8. Procédé suivant la revendication 7, dans lequel la solution colloïdale contient 0,9 à 1,1 g d'ammoniaque par gramme de citrate de bismuth.

9. Procédé suivant les revendications 4 à 8, dans lequel la solution colloïdale contient jusqu'à 44% (p/v) de citrate de bismuth.

10. Procédé suivant la revendication 9, dans lequel la solution colloïdale contient 16 à 30% (p/v) de citrate de bismuth.

11. Procédé suivant la revendication 10, dans lequel la solution colloïdale contient 20 à 25% (p/v) de citrate de bismuth.

12. Procédé suivant les revendications 4, 5, 6 ou 9, dans lequel la solution colloïdale contient 2 à 33% (p/v) d'ammoniaque.

13. Procédé suivant les revendications 4, 5, 6 ou 9, dans lequel la solution colloïdale contient 8 à 33% (p/v) d'ammoniaque.

14. Procédé suivant les revendications 4, 5, 6 ou 9, dans lequel la solution colloïdale contient 20 à 25% (p/v) d'ammoniaque.

15. Composition pharmaceutique contenant une composition définie dans la revendication 1, 2 ou 3, en une quantité efficace pour le traitement des ulcères peptiques, en combinaison avec un excipient pharmaceutiquement acceptable.

16. Procédé de préparation d'une composition pharmaceutique suivant la revendication 15, caractérisé en ce qu'on combine une composition solid contenant du bismuth suivant la revendication 1, 2 ou 3, en une quantité efficace pour le traitement de l'ulcère peptique, avec un excipient pharmaceutiquement acceptable.

17. Procédé suivant la revendication 16, dans lequel un alcool polyhydroxylé est ajouté à la composition.

## Revendications pour l'Etat Contractant: AT

1. Procédé de préparation d'une composition solide qui est colloïdalement soluble dans l'eau, consistant essentiellement en un complexe d'ions bismuth citrate et hydroxyle et en ammoniac, dans laquelle l'ammoniac est présent en une quantité minimale d'environ 2% en poids, par séchage par pulvérisation d'une solution colloïdale de citrate de bismuth dans de l'ammoniaque aqueuse, caractérisé en ce que la solution à sécher par pulvérisation ne contient pas d'alcool polyhydroxylé.

2. Procédé suivant la revendication 1, dans lequel l'ammoniac est présent dans la composition solide en une quantité maximale d'environ 6% en poids.

3. Procédé suivant la revendication 1 ou 2, qui est exécuté à une température d'environ 200—220°C.

4. Procédé suivant la revendication 1, 2 ou 3, dans lequel la solution colloïdale contient 0,2 à 2 g d'ammoniaque par gramme de citrate de bismuth.

5. Procédé suivant la revendication 4, dans lequel la solution colloïdale contient 0,3 à 1,2 g d'ammoniaque par gramme de citrate de bismuth.

6. Procédé suivant la revendication 5, dans lequel la solution colloïdale contient 0,9 à 1,1 g d'ammoniaque par gramme de citrate de bismuth.

7. Procédé suivant les revendications 1—6, dans lequel la solution colloïdale contient jusqu'à 44% (p/v) de citrate de bismuth.

8. Procédé suivant la revendication 7, dans lequel la solution colloïdale contient 16 à 30% (p/v) de citrate de bismuth.

9. Procédé suivant la revendication 8, dans lequel la solution colloïdale contient 20 à 25% (p/v) de citrate de bismuth.

10. Procédé suivant la revendication 1, 2, 3, 4 ou 7, dans lequel la solution colloïdale contient 2 à 33% (p/v) d'ammoniaque.

11. Procédé suivant la revendication 10, dans lequel la solution colloïdale contient 8 à 33% (p/v) d'ammoniaque.

12. Procédé suivant la revendication 11, dans lequel la solution colloïdale contient 20 à 25% (p/v) d'ammoniaque.

13. Procédé de préparation d'une composition pharmaceutique, qui comprend la combinaison de la composition solide contenant du bismuth obtenue suivant l'une quelconque des revendications 1 à 12, en une quantité efficace pour le traitement des ulcères, avec un excipient pharmaceutiquement acceptable.

14. Procédé de préparation d'une composition pharmaceutique suivant la revendication 13, dans lequel un alcool polyhydroxylé est ajouté à la composition.